# EUROPEAN PATENT SPECIFICATION

(11) **EP 2 303 262 B1**
(45) Date of publication and mention of the grant of the patent: **17.01.2018**
(21) Application number: 09757506.2
(22) Date of filing: 02.06.2009
(51) Int. Cl.: A61K 31/522, A61K 45/06, A61K 31/155, A61K 31/4184, A61K 31/4439, A61K 31/40, A61P 1/16

(54) **DPP-IV INHIBITORS FOR USE IN THE TREATMENT OF NAFLD**
VERWENDUNG VON DPP-IV INHIBITOREN ZUR BEHANDLUNG VON NAFLD
INHIBITEURS DE LA DPP-IV POUR LE TRAITEMENT DE LA STÉATOSE HÉPATIQUE NON ALCOOLIQUE (NAFLD)

(30) Priority: 03.06.2008 EP 08157512; 08.08.2008 US 87349 P; 06.02.2009 EP 09152297; 17.02.2009 US 153074 P
(43) Date of publication of application: 06.04.2011
(73) Proprietor: Boehringer Ingelheim International GmbH, 55216 Ingelheim am Rhein (DE)
(72) Inventor: KLEIN, Thomas, 55216 Ingelheim Am Rhein (DE); MARK, Michael, 55216 Ingelheim Am Rhein (DE); NIESSEN, Heiko, 55216 Ingelheim Am Rhein (DE); THOMAS, Leo, 55216 Ingelheim Am Rhein (DE)
(74) Representative: Simon, Elke Anna Maria
(86) International application number: PCT/EP2009/056722
(87) International publication number: WO 2009/147125

(56) References cited:
- WO-A-03/037327
- WO-A-2004/050658
- WO-A-2005/000848
- WO-A-2005/116014
- WO-A-2006/029769
- WO-A-2006/040625
- WO-A-2007/033350
- WO-A-2007/099345
- WO-A-2007/137107
- WO-A-2007/148185
- WO-A-2008/017670
- WO-A1-2009/022007
- WO-A1-2009/022009
- WO-A2-2004/018468
- THOMAS L ET AL: "(R)-8-(3-amino-piperidin-1-yl)-7-but-2-yn yl-3-methyl-1(4-methyl-quina zolin-2-ylmethyl)-3,7-dihydro-purine-2,6-d ione (BI 1356), a novel xanthine-based dipeptidyl peptidase 4 inhibitor, has a superior potency and longer duration of action compared with other dipeptidyl peptidase-4 inhibitors" JOURNAL OF PHARMACOLOGY AND EXPERIMENTAL THERAPEUTICS, vol. 325, no. 1, 1 April 2008 (2008-04-01), pages 175-182, XP009105508 ISSN: 0022-3565
- YASUDA ET AL: "E3024, 3-but-2-ynyl-5-methyl-2-piperazin-1-yl-3,5 -dihydro-4H-imidazo[ 4,5-d]pyridazin-4-one tosylate, is a novel, selective and competitive dipeptidyl peptidase-IV inhibitor" EUROPEAN JOURNAL OF PHARMACOLOGY, vol. 548, no. 1-3, 24 October 2006 (2006-10-24), pages 181-187, XP005658744 ISSN: 0014-2999
- BALABAN YASEMIN H ET AL: "Dipeptidyl peptidase IV (DDP IV) in NASH patients" ANNALS OF HEPATOLOGY, vol. 6, no. 4, 1 October 2007 (2007-10-01), pages 242-250, XP009108799 ISSN: 1665-2681 cited in the application
- Y-L HE ET AL: "The influence of hepatic impairment on the pharmacokinetics of the dipeptidyl peptidase IV (DPP-4) inhibitor vildagliptin" EUROPEAN JOURNAL OF CLINICAL PHARMACOLOGY, vol. 63, no. 7, 8 May 2007 (2007-05-08), pages 677-686, XP019512942 ISSN: 1432-1041
- MENDES F D ET AL: "Recent advances in the treatment of non-alcoholic fatty liver disease" EXPERT OPINION ON INVESTIGATIONAL DRUGS, vol. 14, no. 1, 1 January 2005 (2005-01-01), pages 29-35, XP002354133 ISSN: 1354-3784

## Description

The present invention relates to a DPP-4 inhibitor of formula (I) 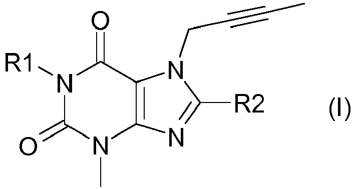 wherein
**R1** denotes (4-methyl-quinazolin-2-yl)methyl, and
**R2** denotes 3-(R)-amino-piperidin-1-yl,
or its pharmaceutically acceptable salt;
for use in the treatment and/or prevention of non alcoholic fatty liver disease (NAFLD).

Reference may be made to the present claims for further dependent or independent embodiments of the invention.

The present description relates to certain DPP-4 inhibitors for treating and/or preventing non alcoholic fatty liver diseases (NAFLD) and to the use of these DPP-4 inhibitors in treating and/or preventing NAFLD and/or diseases related or associated therewith. The description also relates to combinations of certain DPP-4 inhibitors with other active substances for treating and/or preventing NAFLD and to the use of combinations of these DPP-4 inhibitors with other active substances for treating and/or preventing NAFLD. Pharmaceutical compositions for use in the treatment and/or prevention of non alcoholic fatty liver diseases (NAFLD) comprising a DPP-4 inhibitor as defined herein optionally together with one or more other active substances are also contemplated.

The spectrum of fatty liver diseases associated with metabolic determinants and not resulting from alcohol (NAFLD, non-alcoholic fatty liver diseases) extends from simple hepatic steatosis through inflammatory steatohepatitis (NASH, non-alcoholic steatohepatitis) to liver fibrosis and cirrhosis, as well as, in some cases, hepatocellular carcinomas. NASH is the most severe form of NAFLD and recent data indicate prevalences for NASH and NAFLD in the range of 17-33% (in US). Thus, NASH and NAFLD have emerged as clinically important types of chronic liver diseases in industrialized countries (McCullough 2006). The underlying pathophysiological mechanisms of NAFLD and NASH are currently tried to be explained by the first and second hit theory. As a primary event hepatic steatosis is emerging due to either extra-hepatic (e.g. increased free fatty acid influx, insulin resistance, fasting, low levels of adiponectin) or intra-hepatic (e.g. decreased capacity to secrete VLDL, decreased β-oxidation) disturbances. The first hit leads to hepatic steatosis and predisposes and triggers the second hit for an inflammatory event. In this context, increased levels of reactive oxygen species (ROS) and elevated levels of pro-inflammatory cytokines like TNF-a are discussed to promote inflammation. A significant role for the development of NASH and NAFLD accords to the insulin resistance of obese and diabetic individuals. Thus, current therapies focus on break through strategies of insulin resistance by the mean of insulin sensitizers like TZD (thiazolidinediones) or metformin. There are some encouraging results from clinical pilot studies using pioglitazone (Promat 2004) of rosiglitazone (Neuschwander 2003) as well as metformin (Zhou 2001). However, TZD therapy is associated with massive weight gain and fat redistribution. In addition, TZD cause fluid retention and are not indicated in patients with congestive heart failure. Long term treatment with TZD are further associated with an increased risk of bone fractures. Other therapeutic approaches to NASH and NAFLD are rather conservative comprising diet and exercise and weight control. Further, NAFLD and particularly NASH is also associated with an increased risk for endothelial dysfunction and cardiovascular risk, especially over long term. Thus, there is still a high unmet need and a high demand of novel and efficacious medicaments for the treatment of NAFLD and NASH. In a recent publication in the Annals of Hepatology (2007, 6, 242-250) Balaban et al. focused on the role of DPP-4 expression in NASH patients and correlated DPP-4 intensity of immunostaining with histopathological grade of hepatosteatosis and thus, providing additional evidence for the use of DPP-4 inhibitors in this indication.

The enzyme DPP-4 also known as CD26 is a serine protease known to lead to the cleavage of a dipeptide from the N-terminal end of a number of proteins having at their N-terminal end a prolin or alanin residue. Due to this property DPP-4 inhibitors interfere with the plasma level of bioactive peptides including the peptide GLP-1 and are considered to be promising drugs for the treatment of diabetes mellitus.

For example, DPP-4 inhibitors and their uses are disclosed in WO 2002/068420, WO 2004/018467, WO 2004/018468, WO 2004/018469, WO 2004/041820, WO 2004/046148, WO 2005/051950, WO 2005/082906, WO 2005/063750, WO 2005/085246, WO 2006/027204, WO 2006/029769 or WO2007/014886; or in WO 2004/050658, WO 2004/111051, WO 2005/058901 or WO 2005/097798; or in WO 2006/068163, WO 2007/071738 or WO 2008/017670; or in WO 2007/128721 or WO 2007/128761.

As further DPP-4 inhibitors the following compounds can be mentioned:
- Sitagliptin (MK-0431) having the structural formula A below is (3*R*)-3-amino-1-[3-(trifluoromethyl)-5,6,7,8-tetrahydro-5H-[1,2,4]triazolo[4,3-a]pyrazin-7-yl]-4-(2,4,5-trifluorophenyl)butan-1-one, also named (2*R*)-4-oxo-4-[3-(trifluoromethyl)-5,6-dihydro[1,2,4]triazolo[4,3-a]pyrazin-7(8*H*)-yl]-1-(2,4,5-trifluorophenyl)butan-2-amine, 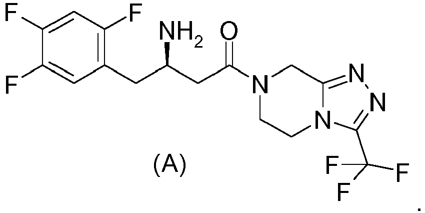
   In one embodiment, sitagliptin is in the form of its dihydrogenphosphate salt, i.e. sitagliptin phosphate. In a further embodiment, sitagliptin phosphate is in the form of a crystalline anhydrate or monohydrate. A class of this embodiment refers to sitagliptin phosphate monohydrate. Sitagliptin free base and pharmaceutically acceptable salts thereof are disclosed in US Patent No. 6,699,871 and in Example 7 of WO 03/004498. Crystalline sitagliptin phosphate monohydrate is disclosed in WO 2005/003135 and in WO 2007/050485.
   For details, e.g. on a process to manufacture, to formulate or to use this compound or a salt thereof, reference is thus made to these documents.
   A tablet formulation for sitagliptin is commercially available under the trade name Januvia®. A tablet formulation for sitagliptin/metformin combination is commercially available under the trade name Janumet®.
- Vildagliptin (LAF-237) having the structural formula B below is (2S)-{[(3-hydroxyadamantan-1-yl)amino]acetyl}pyrrolidine-2-carbonitrile, also named (S)-1-[(3-hydroxy-1-adamantyl)amino]acetyl-2-cyano-pyrrolidine, 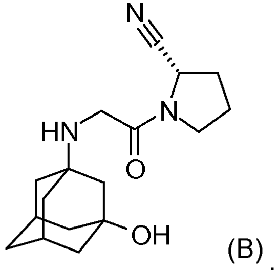
   Vildagliptin is specifically disclosed in US Patent No. 6,166,063 and in Example 1 of WO 00/34241. Specific salts of vildagliptin are disclosed in WO 2007/019255. A crystalline form of vildagliptin as well as a vildagliptin tablet formulation are disclosed in WO 2006/078593. Vildagliptin can be formulated as described in WO 00/34241 or in WO 2005/067976. A modified release vildagliptin formulation is described in WO 2006/135723.
   For details, e.g. on a process to manufacture, to formulate or to use this compound or a salt thereof, reference is thus made to these documents.
   A tablet formulation for vildagliptin is commercially available under the trade name Galvus®. A tablet formulation for vildagliptin/metformin combination is commercially available under the trade name Eucreas®.
- Saxagliptin (BMS-477118) having the structural formula C below is (1S,3S,5S)-2-{(2S)-2-amino-2-(3-hydroxyadamantan-1-yl)acetyl}-2-azabicyclo[3.1.0]hexane-3-carbonitrile, also named (S)-3-hydroxyadamantylglycine-L-*cis*-4,5-methanoprolinenitrile, 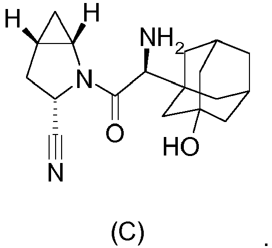
   Saxagliptin is specifically disclosed in US Patent No. 6,395,767 and in Example 60 of WO 01/68603.
   In one embodiment, saxagliptin is in the form of its HCl salt or its mono-benzoate salt as disclosed in WO 2004/052850. In a further embodiment, saxagliptin is in the form of the free base. In a yet further embodiment, saxagliptin is in the form of the monohydrate of the free base as disclosed in WO 2004/052850. Crystalline forms of the HCl salt and the free base of saxagliptin are disclosed in WO 2008/131149. A process for preparing saxagliptin is also disclosed in WO 2005/106011 and WO 2005/115982. Saxagliptin can be formulated in a tablet as described in WO 2005/117841.
   For details, e.g. on a process to manufacture, to formulate or to use this compound or a salt thereof, reference is thus made to these documents.
- Alogliptin (SYR-322) having the structural formula E below is 2-({6-[(3R)-3-aminopiperidin-1-yl]-3-methyl-2,4-dioxo-3,4-dihydro-2H-pyrimidin-1-yl}methyl)benzonitrile 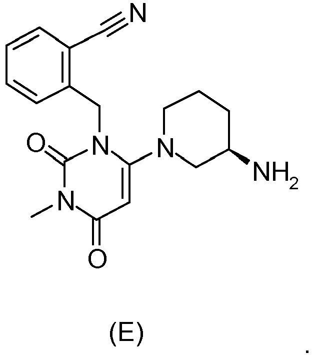
   Alogliptin is specifically disclosed in US 2005/261271, EP 1586571 and in WO 2005/095381. In one embodiment, alogliptin is in the form of its benzoate salt, its hydrochloride salt or its tosylate salt each as disclosed in WO 2007/035629. A class of this embodiment refers to alogliptin benzoate. Polymorphs of alogliptin benzoate are disclosed in WO 2007/035372. A process for preparing alogliptin is disclosed in WO 2007/112368 and, specifically, in WO 2007/035629. Alogliptin (namely its benzoate salt) can be formulated in a tablet and administered as described in WO 2007/033266. Formulations of Aloglipitin with metformin or pioglitazone are described in WO 2008/093882 or WO 2009/011451, respectively.
   For details, e.g. on a process to manufacture, to formulate or to use this compound or a salt thereof, reference is thus made to these documents.
- (2*S*)-1-{[2-(5-Methyl-2-phenyl-oxazol-4-yl)-ethylamino]-acetyl}-pyrrolidine-2-carbonitrile or a pharmaceutically acceptable salt thereof, preferably the mesylate, or (2*S*)-1-{[1,1,-Dimethyl-3-(4-pyridin-3-yl-imidazol-1-yl)-propylamino]-acetyl}-pyrrolidine-2-carbonitrile or a pharmaceutically acceptable salt thereof.
   These compounds and methods for their preparation are disclosed in WO 03/037327.
   The mesylate salt of the former compound as well as crystalline polymorphs thereof are disclosed in WO 2006/100181. The fumarate salt of the latter compound as well as crystalline polymorphs thereof are disclosed in WO 2007/071576. These compounds can be formulated in a pharmaceutical composition as described in WO 2007/017423.
   For details, e.g. on a process to manufacture, to formulate or to use these compounds or salts thereof, reference is thus made to these documents.
- (*S*)-1-((2*S*,3S,11b*S*)-2-Amino-9,10-dimethoxy-1,3,4,6,7,11b-hexahydro-2H-pyrido[2,1-a]isoquinolin-3-yl)-4-fluoromethyl-pyrrolidin-2-one or a pharmaceutically acceptable salt thereof: 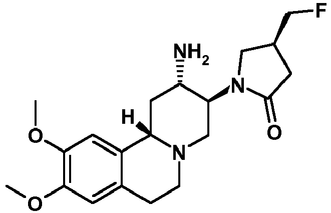
   This compound and methods for its preparation are disclosed in WO 2005/000848. A process for preparing this compound (specifically its dihydrochloride salt) is also disclosed in WO 2008/031749, WO 2008/031750 and WO2008/055814. This compound can be formulated in a pharmaceutical composition as described in WO 2007/017423.
   For details, e.g. on a process to manufacture, to formulate or to use this compound or a salt thereof, reference is thus made to these documents.
- (3,3-Difluoropyrrolidin-1-yl)-((2S,4S)-4-(4-(pyrimidin-2-yl)piperazin-1-yl)pyrrolidin-2-yl)methanone or a pharmaceutically acceptable salt thereof.
   This compound and methods for its preparation are disclosed in WO 2005/116014 and US 7291618.
   For details, e.g. on a process to manufacture, to formulate or to use this compound or a salt thereof, reference is thus made to these documents.
- (1((3S,4S)-4-amino-1-(4-(3,3-difluoropyrrolidin-1-yl)-1,3,5-triazin-2-yl)pyrrolidin-3-yl)-5,5-difluoropiperidin-2-one or a pharmaceutically acceptable salt thereof: 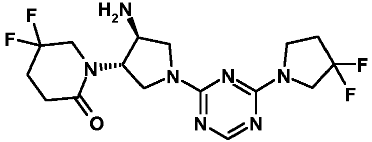
   This compound and methods for its preparation are disclosed in WO 2007/148185 and US 20070299076. For details, e.g. on a process to manufacture, to formulate or to use this compound or a salt thereof, reference is thus made to these documents.
- (2S,4S)-1-{2-[(3S,1R)-3-(1H-1,2,4-Triazol-1-ylmethyl)cyclopentylamino]-acetyl}-4-fluoropyrrolidine-2-carbonitrile or a pharmaceutically acceptable salt thereof: 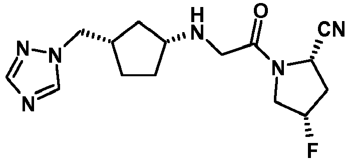
   This compound and methods for its preparation are disclosed in WO 2006/040625 and WO 2008/001195. Specifically claimed salts include the methanesulfonate and p-toluenesulfonate. For details, e.g. on a process to manufacture, to formulate or to use this compound or a salt thereof, reference is thus made to these documents.
- (R)-2-[6-(3-Amino-piperidin-1-yl)-3-methyl-2,4-dioxo-3,4-dihydro-2H-pyrimidin-1-ylmethyl]-4-fluoro-benzonitrile or a pharmaceutically acceptable salt thereof: 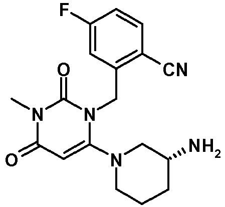
   This compound and methods for its preparation and use are disclosed in WO 2005/095381, US 2007060530, WO 2007/033350, WO 2007/035629, WO 2007/074884, WO 2007/112368 and WO 2008/033851. Specifically claimed salts include the succinate (WO 2008/067465), benzoate, benzenesulfonate, p-toluenesulfonate, (R)-mandelate and hydrochloride. For details, e.g. on a process to manufacture, to formulate or to use this compound or a salt thereof, reference is thus made to these documents.
- 5-{(S)-2-[2-((S)-2-Cyano-pyrroildin-1-yl)-2-oxo-ethylamino]-propyl}-5-(1H-tetrazol-5-yl)-10,11-dihydro-5H-dibenzo[a,d]cycloheptene-2,8-dicarboxylic acid bis-dimethylamide or a pharmaceutically acceptable salt thereof:

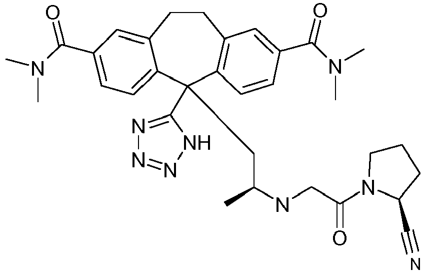

This compound and methods for its preparation are disclosed in WO 2006/116157 and US 2006/270701. For details, e.g. on a process to manufacture, to formulate or to use this compound or a salt thereof, reference is thus made to these documents.

Within the scope of the present description it has now surprisingly been found that DPP-4 inhibitors as defined herein have surprising and particularly advantageous properties, which make them particularly suitable for treating and/or preventing (including preventing, slowing, delaying and/or reversing the progression or reducing the occurrence or delaying the onset) of non alcoholic fatty liver disease (NAFLD) including hepatic steatosis, non-alcoholic steatohepatitis (NASH) and/or liver fibrosis, and/or diseases related therewith (such as e.g. diabetes, insulin insensitivity, hepatic glucose overproduction and/or metabolic syndrome) or associated therewith (such as e.g. cardiovascular disease and/or atherosclerosis or other (cardio)metabolic disorders), and thus for preventing liver cirrhosis (irreversible advanced scarring of the liver) and/or hepatocellular carcinomas.

Thus, the present description provides a DPP-4 inhibitor as defined herein for use in the treatment and/or prevention of non alcoholic fatty liver disease (NAFLD).

In addition, the present description further provides a DPP-4 inhibitor as defined herein for use in improving insulin sensitivity.

The present description further provides a DPP-4 inhibitor as defined herein for use in decreasing glucose production in the liver.

The present description further provides a DPP-4 inhibitor as defined herein for use in the treatment and/or prevention of insulin resistance and/or insulin resistance syndrome.

The present description further provides a DPP-4 inhibitor as defined herein for use in the treatment and/or prevention of metabolic syndrome.

The present description further provides a DPP-4 inhibitor as defined herein for use in the treatment and/or prevention of one or more of the features associated with the metabolic syndrome, such as e.g. NAFLD/NASH, (central) obesity, diabetes, dyslipidemia, hypertension and/or atherosclerotic (cardio)vascular disease or damages.

The present description further provides a DPP-4 inhibitor as defined herein for use in preventing and/or reducing the risk of adverse effects associated with existing therapies of NAFLD/NASH (e.g. using TZD and/or metformin).

Further, the present description further provides a DPP-4 inhibitor as defined herein for reducing the risk of endothelial dysfunction and/or cardiovascular disease or events, e.g. while treating NAFLD/NASH.

Also, the present description provides a DPP-4 inhibitor as defined herein for reducing body weight or preventing an increase in body weight or facilitating a reduction in body weight or positively influencing body fat distribution.

The present description further provides a DPP-4 inhibitor as defined herein for use in the treatment and/or prevention of obesity, especially of severe or extreme obesity (e.g. class II or III), e.g. with a BMI > 35 or even > 40 kg/m² (or ≥ 30 kg/m² for Japanese patients), particularly abdominal and/or visceral obesity.

The present description further provides a DPP-4 inhibitor as defined herein for treating and/or preventing (including reducing the risk of developing or progressing) NAFLD/NASH and/or diseases or disorders related or associated therewith, particularly in patients with or at risk of NAFLD/NASH, such as e.g. those patients having one or more disorders selected from NAFLD, metabolic syndrome, insulin resistance, IGT, IFG, overweight, obesity (e.g. BMI ≥ 25-30 kg/m², waist circumference > 88 (women) - 102 (men) cm, and/or waist:hip ratio > 0.85 (women) - 0.9 (men), particularly visceral and/or abdominal obesity), dyslipidemia (including hyperlipidemia, particularly hypertriglyceridemia (e.g. blood triglyceride level 150 mg/dL) and/or hypoHDLemia (e.g. blood HDL cholesterol level < 40 (men) - 50 (women) mg/dL), diabetes (particularly type 2 diabetes), hypertension (e.g. ≥130/≥85 mmHg), hyperglycemia, hyperinsulinemia, hyperuricemia, severe sleep apnoe, polycystic ovary syndrome, and chronic hepatitis C infection; particularly including elderly patients >45 years.

The present description further provides a DPP-4 inhibitor as defined herein for treating and/or preventing (including reducing the risk of developing or progressing) metabolic disorders or diseases, especially diabetes (particularly type 2 diabetes), in patients having NAFLD/NASH.

Further, according to another aspect of the description, there is provided the use of a DPP-4 inhibitor as defined herein for the manufacture of a medicament for one or more of the following purposes:
- preventing, slowing the progression of, delaying or treating a metabolic disorder or disease, such as e.g. type 1 diabetes mellitus, type 2 diabetes mellitus, impaired glucose tolerance (IGT), impaired fasting blood glucose (IFG), hyperglycemia, postprandial hyperglycemia, overweight, obesity, dyslipidemia, hyperlipidemia, hypercholesterolemia, hypertension, atherosclerosis, endothelial dysfunction, osteoporosis, chronic systemic inflammation, retinopathy, neuropathy, nephropathy and/or metabolic syndrome;
- improving glycemic control and/or for reducing of fasting plasma glucose, of postprandial plasma glucose and/or of glycosylated hemoglobin HbA1c;
- preventing, slowing, delaying or reversing progression from impaired glucose tolerance (IGT), impaired fasting blood glucose (IFG), insulin resistance and/or from metabolic syndrome to type 2 diabetes mellitus;
- preventing, reducing the risk of, slowing the progression of, delaying or treating of complications of diabetes mellitus such as micro- and macrovascular diseases, such as nephropathy, retinopathy, neuropathy, cardio- or cerebrovascular diseases, tissue ischaemia, diabetic foot or ulcus, atherosclerosis, myocardial infarction, acute coronary syndrome, unstable angina pectoris, stable angina pectoris, peripheral arterial occlusive disease, cardiomyopathy, heart failure, heart rhythm disorders, vascular restenosis, and/or stroke;
- reducing body weight or preventing an increase in body weight or facilitating a reduction in body weight;
- preventing, slowing, delaying or treating the degeneration of pancreatic beta cells and/or the decline of the functionality of pancreatic beta cells and/or for improving and/or restoring the functionality of pancreatic beta cells and/or stimulating and/or restoring the functionality of pancreatic insulin secretion;
- for reducing the risk for adverse effects associated with conventional (oral) antihyperglycemic medication; and/or
- maintaining and/or improving the insulin sensitivity and/or for treating or preventing hyperinsulinemia and/or insulin resistance;
particularly in a patient having NAFLD, optionally in combination with one or more other active substances, such as e.g. any of those mentioned herein.

In addition, the present description further provides a DPP-4 inhibitor as defined herein for use in the treatment and/or prevention of fibrosis, such as e.g. fibrosis of the liver, lung, skin, heart or kidney.

The present description further provides the use of a DPP-4 inhibitor as defined herein for the manufacture of a pharmaceutical composition for treatment and/or prevention of those diseases given hereinabove or hereinbelow, particularly non alcoholic fatty liver disease (NAFLD).

The present description further provides a fixed or non-fixed combination including a kit-of-parts for use in the treatment and/or prevention of those diseases given hereinabove, particularly non alcoholic fatty liver disease (NAFLD), said combination comprising a DPP-4 inhibitor as defined herein and optionally one or more other active substances, e.g. any of those mentioned herein, particularly metformin or pioglitazone or an ARB (such as e.g. telmisartan).

The present description further provides the use of a DPP-4 inhibitor as defined herein in combination with one or more other active substances, such as e.g. any of those mentioned herein, particularly metformin or pioglitazone or an ARB (such as e.g. telmisartan), for the manufacture of a pharmaceutical composition for treatment and/or prevention of those diseases given hereinabove, particularly non alcoholic fatty liver disease (NAFLD).

The present description further provides a pharmaceutical composition for use in the treatment and/or prevention of those diseases given hereinabove, particularly non alcoholic fatty liver disease (particularly diabetic NAFLD), said pharmaceutical composition comprising a DPP-4 inhibitor as defined herein and optionally one or more other active substances, such as e.g. any of those mentioned herein, particularly metformin or pioglitazone or an ARB (such as e.g. telmisartan), such as e.g. for separate, sequential, simultaneous, concurrent or chronologically staggered use of the active ingredients.

The present description further provides a pharmaceutical composition for use in the treatment and/or prevention of those diseases given hereinabove, particularly non alcoholic fatty liver disease (NAFLD), said pharmaceutical composition comprising a DPP-4 inhibitor as defined herein as sole active ingredient or, optionally, together with one or more other active substances, such as e.g. any of those mentioned herein, particularly metformin or pioglitazone or an ARB (such as e.g. telmisartan), and, optionally, one or more pharmaceutically acceptable carriers and/or diluents.

The present description further provides a method of treating and/or preventing any of those diseases given hereinabove, particularly non alcoholic fatty liver disease (NAFLD), said method comprising administering to a subject in need thereof (particularly a human patient) an effective amount of a DPP-4 inhibitor as defined herein, optionally alone or in combination, such as e.g. separately, sequentially, simultaneously, concurrently or chronologically staggered with an effective amount of one, two or more other active substances, such as e.g. any of those mentioned herein, particularly metformin or pioglitazone or an ARB (such as e.g. telmisartan).

In an embodiment of this invention, non alcoholic fatty liver disease (NAFLD) within the meaning of this invention includes, without being limited to hepatic steatosis, non-alcoholic steatohepatitis (NASH) and/or liver fibrosis.

In a special embodiment of this invention, non alcoholic fatty liver disease (NAFLD) within the meaning of this invention refers to non-alcoholic steatohepatitis (NASH).

In an embodiment of this invention, the therapies of this invention provide improvements in one or more histopathological features of patients, such as e.g. in portal inflammation, hepatosteatosis, ballooning degeneration, and/or lobular inflammation.

A DPP-4 inhibitor within the meaning of the present description include, without being limited to, any of those DPP-4 inhibitors mentioned hereinabove and hereinbelow, preferably orally active DPP-4 inhibitors.

In a first embodiment (embodiment **A**), a DPP-4 inhibitor in the context of the present description is any DPP-4 inhibitor of formula (I)
or formula (II) 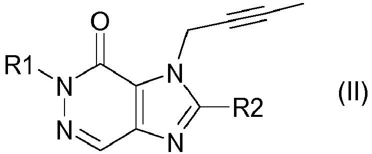 or formula (III) 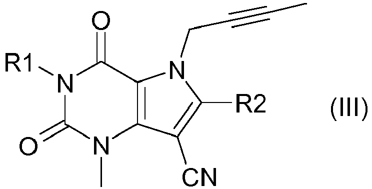 or formula (IV) 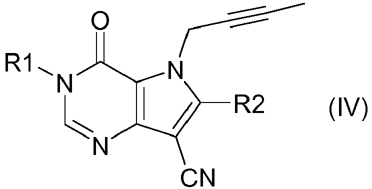 wherein **R1** denotes ([1,5]naphthyridin-2-yl)methyl, (quinazolin-2-yl)methyl, (quinoxalin-6-yl)methyl, (4-methyl-quinazolin-2-yl)methyl, 2-cyano-benzyl, (3-cyano-quinolin-2-yl)methyl, (3-cyano-pyridin-2-yl)methyl, (4-methyl-pyrimidin-2-yl)methyl, or (4,6-dimethyl-pyrimidin-2-yl)methyl and **R2** denotes 3-(*R*)-amino-piperidin-1-yl, (2-amino-2-methyl-propyl)-methylamino or (2-(*S*)-amino-propyl)-methylamino,
or its pharmaceutically acceptable salt.

In a second embodiment (embodiment **B**), a DPP-4 inhibitor in the context of the present description is a DPP-4 inhibitor selected from the group consisting of sitagliptin, vildagliptin, saxagliptin, alogliptin,
(2*S*)-1-{[2-(5-Methyl-2-phenyl-oxazol-4-yl)-ethylamino]-acetyl}-pyrrolidine-2-carbonitrile, (2*S*)-1-{[1,1,-Dimethyl-3-(4-pyridin-3-yl-imidazol-1-yl)-propylamino]-acetyl}-pyrrolidine-2-carbonitrile,
(*S*)-1-((2*S*,3*S*,11b*S*)-2-Amino-9,10-dimethoxy-1,3,4,6,7,11b-hexahydro-2H-pyrido[2,1-a]isoquinolin-3-yl)-4-fluoromethyl-pyrrolidin-2-one,
(3,3-Difluoropyrrolidin-1-yl)-((2S,4S)-4-(4-(pyrimidin-2-yl)piperazin-1-yl)pyrrolidin-2-yl)methanone,
(1((3S,4S)-4-amino-1-(4-(3,3-difluoropyrrolidin-1-yl)-1,3,5-triazin-2-yl)pyrrolidin-3-yl)-5,5-difluoropiperidin-2-one,
(2S,4S)-1-{2-[(3S,1R)-3-(1H-1,2,4-Triazol-1-ylmethyl)cyclopentylamino]-acetyl}-4-fluoropyrrolidine-2-carbonitrile,
(R)-2-[6-(3-Amino-piperidin-1-yl)-3-methyl-2,4-dioxo-3,4-dihydro-2H-pyrimidin-1-ylmethyl]-4-fluoro-benzonitrile, and
5-{(S)-2-[2-((S)-2-Cyano-pyrrolidin-1-yl)-2-oxo-ethylamino]-propyl}-5-(1H-tetrazol-5-yl)-10,11-dihydro-5H-dibenzo[a,d]cycloheptene-2,8-dicarboxylic acid bis-dimethylamide,
or its pharmaceutically acceptable salt.

Regarding the first embodiment (embodiment **A**), preferred DPP-4 inhibitors are any or all of the following compounds and their pharmaceutically acceptable salts:
- 1-[(4-methyl-quinazolin-2-yl)methyl]-3-methyl-7-(2-butyn-1-yl)-8-(3-(*R*)-amino-piperidin-1-yl)-xanthine (compare WO 2004/018468, example 2(142)): 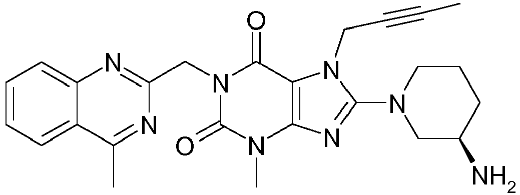
- 1-[([1,5]naphthyridin-2-yl)methyl]-3-methyl-7-(2-butyn-1-yl)-8-((*R*)-3-amino-piperidin-1-yl)-xanthine (compare WO 2004/018468, example 2(252)): 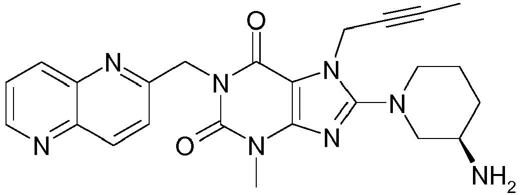
- 1-[(Quinazolin-2-yl)methyl]-3-methyl-7-(2-butyn-1-yl)-8-((*R*)-3-amino-piperidin-1-yl)-xanthine (compare WO 2004/018468, example 2(80)): 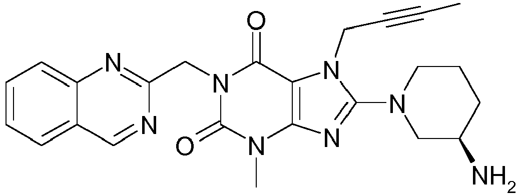
- 2-((R)-3-Amino-piperidin-1-yl)-3-(but-2-yinyl)-5-(4-methyl-quinazolin-2-ylmethyl)-3,5-dihydro-imidazo[4,5-d]pyridazin-4-one (compare WO 2004/050658, example 136): 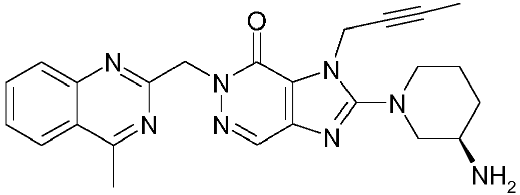
- 1-[(4-Methyl-quinazolin-2-yl)methyl]-3-methyl-7-(2-butyin-1-yl)-8-[(2-amino-2-methylpropyl)-methylamino]-xanthine (compare WO 2006/029769, example 2(1)): 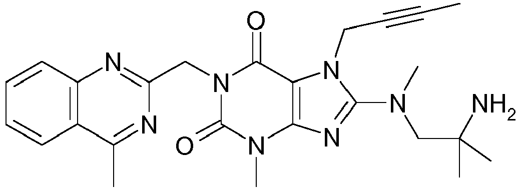
- 1-[(3-Cyano-quinolin-2-yl)methyl]-3-methyl-7-(2-butyn-1-yl)-8-((*R*)-3-amino-piperidin-1-yl)-xanthine (compare WO 2005/085246, example 1(30)): 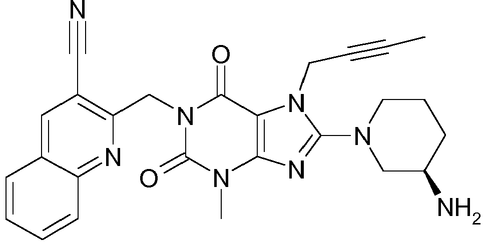
- 1-(2-Cyano-benzyl)-3-methyl-7-(2-butyn-1-yl)-8-((*R*)-3-amino-piperidin-1-yl)-xanthine (compare WO 2005/085246, example 1(39)): 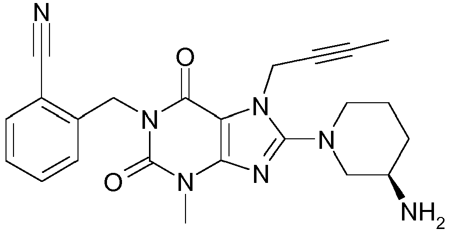
- 1-[(4-Methyl-quinazolin-2-yl)methyl]-3-methyl-7-(2-butyn-1-yl)-8-[(*S*)-(2-amino-propyl)-methylamino]-xanthine (compare WO 2006/029769, example 2(4)): 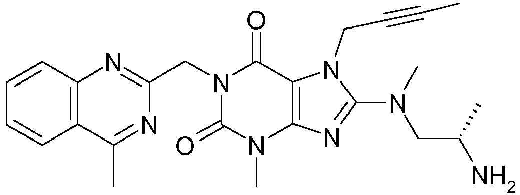
- 1-[(3-Cyano-pyridin-2-yl)methyl]-3-methyl-7-(2-butyn-1-yl)-8-((*R*)-3-amino-piperidin-1-yl)-xanthine (compare WO 2005/085246, example 1(52)): 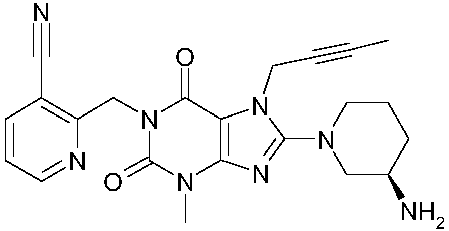
- 1-[(4-Methyl-pyrimidin-2-yl)methyl]-3-methyl-7-(2-butyn-1-yl)-8-((*R*)-3-amino-piperidin-1-yl)-xanthine (compare WO 2005/085246, example 1(81)): 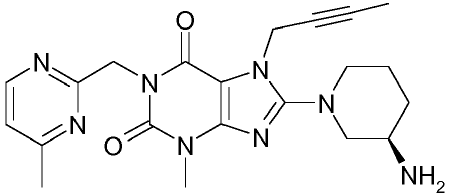
- 1-[(4,6-Dimethyl-pyrimidin-2-yl)methyl]-3-methyl-7-(2-butyn-1-yl)-8-((*R*)-3-amino-piperidin-1-yl)-xanthine (compare WO 2005/085246, example 1(82)): 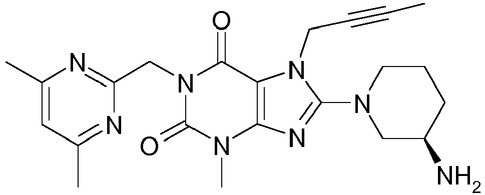
- 1-[(Quinoxalin-6-yl)methyl]-3-methyl-7-(2-butyn-1-yl)-8-((*R*)-3-amino-piperidin-1-yl)-xanthine (compare WO 2005/085246, example 1(83)):

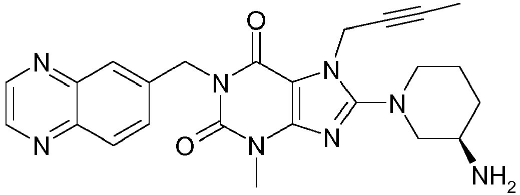

These DPP-4 inhibitors are distinguished from structurally comparable DPP-4 inhibitors, as they combine exceptional potency and a long-lasting effect with favourable pharmacological properties, receptor selectivity and a favourable side-effect profile or bring about unexpected therapeutic advantages or improvements when combined with other pharmaceutical active substances. Their preparation is disclosed in the publications mentioned.

A more preferred DPP-4 inhibitor among the abovementioned DPP-4 inhibitors of embodiment **A** of this description is 1-[(4-methyl-quinazolin-2-yl)methyl]-3-methyl-7-(2-butyn-1-yl)-8-(3-(R)-amino-piperidin-1-yl)-xanthine, particularly the free base thereof (which is also known as Bl 1356).

Regarding the second embodiment (embodiment **B**), preferred DPP-4 inhibitors are selected from the group consisting of saxagliptin and alogliptin, and their pharmaceutically acceptable salts.

Unless otherwise noted, according to this description it is to be understood that the definitions of the active compounds (including the DPP-4 inhibitors) mentioned hereinabove and hereinbelow also comprise their pharmaceutically acceptable salts as well as hydrates, solvates and polymorphic forms thereof. With respect to salts, hydrates and polymorphic forms thereof, particular reference is made to those which are referred to herein.

With respect to embodiment **A,** the methods of synthesis for the DPP-4 inhibitors according to embodiment **A** of this description are known to the skilled person. Advantageously, the DPP-4 inhibitors according to embodiment **A** of this description can be prepared using synthetic methods as described in the literature. Thus, for example, purine derivatives of formula (I) can be obtained as described in WO 2002/068420, WO 2004/018468, WO 2005/085246, WO 2006/029769 or WO 2006/048427.
Purine derivatives of formula (II) can be obtained as described, for example, in WO 2004/050658 or WO 2005/110999.
Purine derivatives of formula (III) and (IV) can be obtained as described, for example, in WO 2006/068163, WO 2007/071738 or WO 2008/017670. The preparation of those DPP-4 inhibitors, which are specifically mentioned hereinabove, is disclosed in the publications mentioned in connection therewith. Polymorphous crystal modifications and formulations of particular DPP-4 inhibitors are disclosed in WO 2007/128721 and WO 2007/128724, respectively. Formulations of particular DPP-4 inhibitors with metformin or other combination partners are described in PCT/EP2009053978. Typical dosage strengths of the dual combination of Bl 1356 / metformin are 2.5/500 mg, 2.5/850 mg and 2.5/1000 mg, each of which may be administered orally once or twice daily, in particular twice daily.

With respect to embodiment **B,** the methods of synthesis for the DPP-4 inhibitors of embodiment **B** are described in the scientific literature and/ or in published patent documents, particularly in those cited herein.

For pharmaceutical application in warm-blooded vertebrates, particularly humans, the compounds of this description are usually used in dosages from 0.001 to 100 mg/kg body weight, preferably at 0.1-15 mg/kg, in each case 1 to 4 times a day. For this purpose, the compounds, optionally combined with other active substances, may be incorporated together with one or more inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, cetylstearyl alcohol, carboxymethylcellulose or fatty substances such as hard fat or suitable mixtures thereof into conventional galenic preparations such as plain or coated tablets, capsules, powders, suspensions or suppositories.

The pharmaceutical compositions according to this description comprising the DPP-4 inhibitors as defined herein are thus prepared by the skilled person using pharmaceutically acceptable formulation excipients as described in the art. Examples of such excipients include, without being restricted to diluents, binders, carriers, fillers, lubricants, flow promoters, crystallisation retardants, disintegrants, solubilizers, colorants, pH regulators, surfactants and emulsifiers.

Examples of suitable diluents for compounds according to embodiment **A** include cellulose powder, calcium hydrogen phosphate, erythritol, low substituted hydroxypropyl cellulose, mannitol, pregelatinized starch or xylitol.

Examples of suitable lubricants for compounds according to embodiment **A** include talc, polyethyleneglycol, calcium behenate, calcium stearate, hydrogenated castor oil or magnesium stearate.

Examples of suitable binders for compounds according to embodiment **A** include copovidone (copolymerisates of vinylpyrrolidon with other vinylderivates), hydroxypropyl methylcellulose (HPMC), hydroxypropylcellulose (HPC), polyvinylpyrrolidon (povidone), pregelatinized starch, or low-substituted hydroxypropylcellulose (L-HPC).

Examples of suitable disintegrants for compounds according to embodiment **A** include corn starch or crospovidone.

Suitable methods of preparing pharmaceutical formulations of the DPP-4 inhibitors according to embodiment **A** of the description are
- direct tabletting of the active substance in powder mixtures with suitable tabletting excipients;
- granulation with suitable excipients and subsequent mixing with suitable excipients and subsequent tabletting as well as film coating; or
- packing of powder mixtures or granules into capsules.

Suitable granulation methods are
- wet granulation in the intensive mixer followed by fluidised bed drying;
- one-pot granulation;
- fluidised bed granulation; or
- dry granulation (e.g. by roller compaction) with suitable excipients and subsequent tabletting or packing into capsules.

For details on dosage forms, formulations and administration of DPP-4 inhibitors of this description, reference is made to scientific literature and/ or published patent documents, particularly to those cited herein.

With respect to the first embodiment (embodiment **A**), the dosage typically required of the DPP-4 inhibitors mentioned herein in embodiment **A** when administered intravenously is 0.1 mg to 10 mg, preferably 0.25 mg to 5 mg, and when administered orally is 0.5 mg to 100 mg, preferably 2.5 mg to 50 mg or 0.5 mg to 10 mg, more preferably 2.5 mg to 10 mg or 1 mg to 5 mg, in each case 1 to 4 times a day. Thus, e.g. the dosage of 1-[(4-methyl-quinazolin-2-yl)methyl]-3-methyl-7-(2-butyn-1-yl)-8-(3-(R)-amino-piperidin-1-yl)-xanthine when administered orally may be 5 mg to 50 mg, 20 mg to 50 mg, 0.5 mg to 10 mg, or 2.5 mg to 10 mg or 1 mg to 5 mg per patient per day.

A dosage form prepared with a pharmaceutical composition comprising a DPP-4 inhibitor mentioned herein in embodiment **A** contain the active ingredient in a dosage range of 0.1-100 mg. Thus, e.g. particular dosage strengths of 1-[(4-methyl-quinazolin-2-yl)methyl]-3-methyl-7-(2-butyn-1-yl)-8-(3-(R)-amino-piperidin-1-yl)-xanthine are 0.5 mg, 1 mg, 2.5 mg, 5 mg and 10 mg.

It will be appreciated that the amount of a compound of this description required for use in treatment and/or prevention may vary, e.g. with the nature and stage of the disease or condition being treated and/or prevented.

With respect to the second embodiment (embodiment **B**), the doses of DPP-4 inhibitors mentioned herein in embodiment **B** to be administered to mammals, for example human beings, of, for example, approximately 70 kg body weight, may be generally from about 0.5 mg to about 350 mg, for example from about 10 mg to about 250 mg, preferably 20-200 mg, more preferably 20-100 mg, of the active moiety per person per day, or from about 0.5 mg to about 20 mg, preferably 2.5-10 mg, per person per day, divided preferably into 1 to 4 single doses which may, for example, be of the same size. Single dosage strengths comprise, for example, 10, 25, 40, 50, 75, 100, 150 and 200 mg of the DPP-4 inhibitor active moiety.

A dosage strength of the DPP-4 inhibitor sitagliptin is usually between 25 and 200 mg of the active moiety. A recommended dose of sitagliptin is 100 mg calculated for the active moiety (free base anhydrate) once daily. Unit dosage strengths of sitagliptin free base anhydrate (active moiety) are 25, 50, 75, 100, 150 and 200 mg. Particular unit dosage strengths of sitagliptin (e.g. per tablet) are 25, 50 and 100 mg. An equivalent amount of sitagliptin phosphate monohydrate to the sitagliptin free base anhydrate is used in the pharmaceutical compositions, namely, 32.13, 64.25, 96.38, 128.5, 192.75, and 257 mg, respectively. Adjusted dosages of 25 and 50 mg sitagliptin are used for patients with renal failure. Typical dosage strengths of the dual combination of sitagliptin / metformin are 50/500 mg and 50/1000 mg, each of which may be administered orally once or twice daily, in particular twice daily.

A dosage range of the DPP-4 inhibitor vildagliptin is usually between 10 and 150 mg daily, in particular between 25 and 150 mg, 25 and 100 mg or 25 and 50 mg or 50 and 100 mg daily. Particular examples of daily oral dosage are 25, 30, 35, 45, 50, 55, 60, 80, 100 or 150 mg. In a more particular aspect, the daily administration of vildagliptin may be between 25 and 150 mg or between 50 and 100 mg. In another more particular aspect, the daily administration of vildagliptin may be 50 or 100 mg. The application of the active ingredient may occur up to three times a day, preferably one or two times a day. Particular dosage strengths are 50 mg or 100 mg vildagliptin. Typical dosage strengths of the dual combination of vildagliptin / metformin are 50/850 mg and 50/1000 mg, each of which may be administered orally once or twice daily, in particular twice daily.

Alogliptin may be administered to a patient at a daily dose of between 5 mg/day and 250 mg/day, optionally between 10 mg and 200 mg, optionally between 10 mg and 150 mg, and optionally between 10 mg and 100 mg of alogliptin (in each instance based on the molecular weight of the free base form of alogliptin). Thus, specific dosage amounts that may be used include, but are not limited to 10 mg, 12.5 mg, 20 mg, 25 mg, 50 mg, 75 mg and 100 mg of alogliptin per day. Alogliptin may be administered in its free base form or as a pharmaceutically acceptable salt.

Saxagliptin may be administered to a patient at a daily dose of between 2.5 mg/day and 100 mg/day, optionally between 2.5 mg and 50 mg. Specific dosage amounts that may be used include, but are not limited to 2.5 mg, 5 mg, 10 mg, 15 mg, 20 mg, 30 mg , 40 mg, 50 mg and 100 mg of saxagliptin per day. Typical dosage strengths of the dual combination of saxagliptin / metformin are 2.5/500 mg and 2.5/1000 mg, each of which may be administered orally once or twice daily, in particular twice daily.

A special embodiment of the DPP-4 inhibitors of this description refers to those orally administered DPP-4 inhibitors which are therapeutically efficacious at low dose levels, e.g. at oral dose levels < 100 mg or < 70 mg per patient per day, preferably < 50 mg, more preferably < 30 mg or < 20 mg, even more preferably from 1 mg to 10 mg, particularly from 1 mg to 5 mg (more particularly 5 mg), per patient per day (if required, divided into 1 to 4 single doses, particularly 1 or 2 single doses, which may be of the same size, preferentially, administered orally once- or twice daily (more preferentially once-daily), advantageously, administered at any time of day, with or without food. Thus, for example, the daily oral amount 5 mg Bl 1356 can be given in a once daily dosing regimen (i.e. 5 mg Bl 1356 once daily) or in a twice daily dosing regimen (i.e. 2.5 mg Bl 1356 twice daily), at any time of day, with or without food.

A particularly preferred DPP-4 inhibitor to be emphasized within the meaning of this invention is 1-[(4-methyl-quinazolin-2-yl)methyl]-3-methyl-7-(2-butyn-1-yl)-8-(3-(*R*)-amino-piperidin-1-yl)-xanthine (also known as Bl 1356). Bl 1356 exhibits high potency, 24h duration of action, and a wide therapeutic window. In people with type 2 diabetes Bl 1356 shows a placebo-like safety and tolerability. With low doses of about ≥ 5 mg, Bl 1356 acts as a true once-daily oral drug with a full 24 h duration of DPP-4 inhibition. At therapeutic oral dose levels, Bl 1356 is mainly excreted via the liver and only to a minor extent (about < 7% of the administered oral dose) via the kidney. Bl 1356 is primarily excreted unchanged via the bile. The fraction of Bl 1356 eliminated via the kidneys increases only very slightly over time and with increasing dose, so that there will likely be no need to modify the dose of Bl 1356 based on the patients' renal function. The non-renal elimination of Bl 1356 in combination with its low accumulation potential and broad safety margin may be of significant benefit in a diabetes patient population that has a high prevalence of renal insufficiency and diabetic nephropathy.

As different metabolic functional disorders often occur simultaneously, it is quite often indicated to combine a number of different active principles with one another. Thus, depending on the functional disorders diagnosed, improved treatment outcomes may be obtained if a DPP-4 inhibitor is combined with active substances customary for the respective disorders, such as e.g. one or more active substances selected from among the other antidiabetic substances, especially active substances that lower the blood sugar level or the lipid level in the blood, raise the HDL level in the blood, lower blood pressure or are indicated in the treatment of atherosclerosis or obesity. Further, within the meaning of this description, optionally in addition, a DPP-4 inhibitor may be combined with one or more antioxidants and/or anti-inflammatory agents. Yet further, within the meaning of this description, optionally in addition, a DPP-4 inhibitor may be combined with one or more vascular endothelial protective agents.

The DPP-4 inhibitors mentioned above - besides their use in mono-therapy - may also be used in conjunction with other active substances, by means of which improved treatment results can be obtained. Such a combined treatment may be given as a free combination of the substances or in the form of a fixed combination, for example in a tablet or capsule. Pharmaceutical formulations of the combination partner(s) needed for this may either be obtained commercially as pharmaceutical compositions or may be formulated by the skilled man using conventional methods. The active substances which may be obtained commercially as pharmaceutical compositions are described in numerous places in the prior art, for example in the list of drugs that appears annually, the "Rote Liste ®" of the federal association of the pharmaceutical industry, or in the annually updated compilation of manufacturers' information on prescription drugs known as the "Physicians' Desk Reference".

Examples of antidiabetic combination partners are metformin; sulphonylureas such as glibenclamide, tolbutamide, glimepiride, glipizide, gliquidon, glibornuride and gliclazide; nateglinide; repaglinide; thiazolidinediones such as rosiglitazone and pioglitazone; PPAR gamma modulators such as metaglidases; PPAR-gamma agonists such as Gl 262570; PPAR-gamma antagonists; PPAR-gamma/alpha modulators such as tesaglitazar, muraglitazar and KRP297; PPAR-gamma/alpha/delta modulators; AMPK-activators such as AlCAR; acetyl-CoA carboxylase (ACC1 and ACC2) inhibitors; diacylglycerol-acetyltransferase (DGAT) inhibitors; pancreatic beta cell GCRP agonists such as SMT3-receptor-agonists and GPR119; 11β-HSD-inhibitors; FGF19 agonists or analogues; alpha-glucosidase blockers such as acarbose, voglibose and miglitol; alpha2-antagonists; insulin and insulin analogues such as human insulin, insulin lispro, insulin glusilin, r-DNA-insulinaspart, NPH insulin, insulin detemir, insulin zinc suspension and insulin glargin; Gastric inhibitory Peptide (GIP); pramlintide; amylin or GLP-1 and GLP-1 analogues such as Exendin-4, e.g. exenatide, exenatide LAR, liraglutide, taspoglutide or albiglutide; SGLT2-inhibitors such as KGT-1251; inhibitors of protein tyrosine-phosphatase; inhibitors of glucose-6-phosphatase; fructose-1,6-bisphosphatase modulators; glycogen phosphorylase modulators; glucagon receptor antagonists; phosphoenolpyruvatecarboxykinase (PEPCK) inhibitors; pyruvate dehydrogenasekinase (PDK) inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976); glucokinase/regulatory protein modulators incl. glucokinase activators; glycogen synthase kinase inhibitors; inhibitors of the SH2-domain-containing inositol 5-phosphatase type 2 (SHIP2) ; IKK inhibitors such as high-dose salicylate ; JNK1 inhibitors ; protein kinase C-theta inhibitors; beta 3 agonists such as ritobegron, YM 178, solabegron, talibegron, N-5984, GRC-1087, rafabegron, FMP825; aldosereductase inhibitors such as AS 3201, zenarestat, fidarestat, epalrestat, ranirestat, NZ-314, CP-744809, and CT-112; SGLT-1 or SGLT-2 inhibitors; KV 1.3 channel inhibitors; GPR40 modulators; SCD-1 inhibitors; CCR-2 antagonists; dopamine receptor agonists (bromocriptine mesylate/Cycloset); and other DPP IV inhibitors.

A preferred example of an antidiabetic combination partner is metformin, which is usually given in doses varying from about 250 mg to 3000 mg, particularly from 500 mg to 2000 mg up to 2500 mg per day using various dosing regimens, such as e.g. usually in doses of about 100 mg to 500 mg or 200 mg to 850 mg (1-3 times a day), or about 300 mg to 1000 mg once or twice a day, or delayed-release metformin in doses of about 100 mg to 1000 mg or preferably 500 mg to 1000 mg once or twice a day or about 500 mg to 2000 mg once a day. Particular dosage strengths may be 250, 500, 625, 750, 850 and 1000 mg of metformin hydrochloride.

Another preferred example is pioglitazone, usually in a dosage of about 1-10 mg, 15 mg, 30 mg, or 45 mg once a day.

Rosiglitazone is usually given in doses from 4 to 8 mg once (or divided twice) a day (typical dosage strengths are 2, 4 and 8 mg).

Glibenclamide (glyburide) is usually given in doses from 2.5-5 to 20 mg once (or divided twice) a day (typical dosage strengths are 1.25, 2.5 and 5 mg), or micronized glibenclamide in doses from 0.75-3 to 12 mg once (or divided twice) a day (typical dosage strengths are 1.5, 3, 4.5 and 6 mg).

Glipizide is usually given in doses from 2.5 to 10-20 mg once (up to 40 mg divided twice) a day (typical dosage strengths are 5 and 10 mg), or extended-release glipizide in doses from 5 to 10 mg (up to 20 mg) once a day (typical dosage strengths are 2.5, 5 and 10 mg).

Glimepiride is usually given in doses from 1-2 to 4 mg (up to 8 mg) once a day (typical dosage strengths are 1, 2 and 4 mg).

A dual combination of glibenclamide/metformin is usually given in doses from 1.25/250 once daily to 10/1000 mg twice daily (typical dosage strengths are 1.25/250, 2.5/500 and 5/500 mg).
A dual combination of glipizide/metformin is usually given in doses from 2.5/250 to 10/1000 mg twice daily (typical dosage strengths are 2.5/250, 2.5/500 and 5/500 mg).
A dual combination of glimepiride/metformin is usually given in doses from 1/250 to 4/1000 mg twice daily.
A dual combination of rosiglitazone/glimepiride is usually given in doses from 4/1 once or twice daily to 4/2 mg twice daily (typical dosage strengths are 4/1, 4/2, 4/4, 8/2 and 8/4 mg). A dual combination of pioglitazone/glimepiride is usually given in doses from 30/2 to 30/4 mg once daily (typical dosage strengths are 30/4 and 45/4 mg).
A dual combination of rosiglitazone/metformin is usually given in doses from 1/500 to 4/1000 mg twice daily (typical dosage strengths are 1/500, 2/500, 4/500, 2/1000 and 4/1000 mg).
A dual combination of pioglitazone/metformin is usually given in doses from 15/500 once or twice daily to 15/850 mg thrice daily (typical dosage strengths are 15/500 and 15/850 mg).

The non-sulphonylurea insulin secretagogue nateglinide is usually given in doses from 60 to 120 mg with meals (up to 360 mg/day, typical dosage strengths are 60 and 120 mg); repaglinide is usually given in doses from 0.5 to 4 mg with meals (up to 16 mg/day, typical dosage strengths are 0.5, 1 and 2 mg). A dual combination of repaglinide/metformin is available in dosage strengths of 1/500 and 2/850 mg.

Acarbose is usually given in doses from 25 to 100 mg with meals (up to 300 mg/day, typical dosage strengths are 25, 50 and 100 mg). Miglitol is usually given in doses from 25 to 100 mg with meals (up to 300 mg/day, typical dosage strengths are 25, 50 and 100 mg).

Examples of combination partners that lower the lipid level in the blood are HMG-CoA-reductase inhibitors such as simvastatin, atorvastatin, lovastatin, fluvastatin, pravastatin and rosuvastatin; fibrates such as bezafibrate, fenofibrate, clofibrate, gemfibrozil, etofibrate and etofyllinclofibrate; nicotinic acid and the derivatives thereof such as acipimox; PPAR-alpha agonists; PPAR-delta agonists; inhibitors of acyl-coenzyme A:cholesterolacyltransferase (ACAT; EC 2.3.1.26) such as avasimibe; cholesterol resorption inhibitors such as ezetimib; substances that bind to bile acid, such as cholestyramine, colestipol and colesevelam; inhibitors of bile acid transport; HDL modulating active substances such as D4F, reverse D4F, LXR modulating active substances and FXR modulating active substances; CETP inhibitors such as torcetrapib, JTT-705/dalcetrapib, anacetrapib or compound 12 from WO 2007/005572; LDL receptor modulators; and ApoB100 antisense RNA.

A dosage of the partner drug atorvastatin is usually from 1 mg to 40 mg or 10 mg to 80 mg once a day

Examples of combination partners that lower blood pressure are beta-blockers such as atenolol, bisoprolol, celiprolol, metoprolol and carvedilol; diuretics such as hydrochlorothiazide, chlortalidon, xipamide, furosemide, piretanide, torasemide, spironolactone, eplerenone, amiloride and triamterene; calcium channel blockers such as amlodipine, nifedipine, nitrendipine, nisoldipine, nicardipine, felodipine, lacidipine, lercanipidine, manidipine, isradipine, nilvadipine, verapamil, gallopamil and diltiazem; ACE inhibitors such as ramipril, lisinopril, cilazapril, quinapril, captopril, enalapril, benazepril, perindopril, fosinopril and trandolapril; as well as angiotensin II receptor blockers (ARBs) such as telmisartan, candesartan, valsartan, losartan, irbesartan, olmesartan and eprosartan.

A dosage of the partner drug telmisartan is usually from 20 mg to 320 mg or 40 mg to 160 mg per day.

Examples of combination partners which increase the HDL level in the blood are Cholesteryl Ester Transfer Protein (CETP) inhibitors; inhibitors of endothelial lipase; regulators of ABC1; LXRalpha antagonists; LXRbeta agonists; PPAR-delta agonists; LXRalpha/beta regulators, and substances that increase the expression and/or plasma concentration of apolipoprotein A-I.

Examples of combination partners for the treatment of obesity are sibutramine; tetrahydrolipstatin (orlistat); alizyme; dexfenfluramine; axokine; cannabinoid receptor 1 antagonists such as the CB1 antagonist rimonobant; MCH-1 receptor antagonists; MC4 receptor agonists; NPY5 as well as NPY2 antagonists; beta3-AR agonists such as SB-418790 and AD-9677; 5HT2c receptor agonists such as APD 356; myostatin inhibitors; Acrp30 and adiponectin; steroyl CoA desaturase (SCD1) inhibitors; fatty acid synthase (FAS) inhibitors; CCK receptor agonists; Ghrelin receptor modulators; Pyy 3-36; orexin receptor antagonists; and tesofensine.

Examples of combination partners for the treatment of atherosclerosis are phospholipase A2 inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976); oxLDL antibodies and oxLDL vaccines; apoA-1 Milano; ASA; and VCAM-1 inhibitors.

Examples of antioxidant combination partners are selenium, betaine, vitamin C, vitamin E and beta carotene.

An example of an anti-inflammatory combination partner is pentoxifylline; another example of an anti-inflammatory combination partner is a PDE-4 inhibitor, such as e.g. tetomilast, roflumilast, or3-[7-ethyl-2-(methoxymethyl)-4-(5-methyl-3-pyridinyl)pyrrolo[1,2-b]pyridazin-3-yl]propanoic acid (or other species disclosed in US 7153854, WO 2004/063197, US 7459451 and/or WO 2006/004188).

A further example of an anti-inflammatory partner drug is a caspase inhibitor, such as e.g. (3S)-5-fluoro-3-({[(5R)-5-isopropyl-3-(1-isoquinolinyl)-4,5-dihydro-5-isoxazolyl]carbonyl}amino-4-oxopentanoic acid (or other species disclosed in WO 2005/021516 and/or WO 2006/090997).

An example of a vascular endothelial protective agent is a PDE-5 inhibitor, such as e.g. sildenafil, vardenafil or tadalafil; another example of a vascular endothelial protective agent is a nitric oxide donor.

Further, within the meaning of this description, optionally in addition, a DPP-4 inhibitor may be combined with one or more CCK-2 or gastrin agonists, such as e.g. proton pump inhibitors (including reversible as well as irreversible inhibitors of the gastric H+/K+-ATPase), for example omeprazole, esomeprazole, pantoprazole, rabeprazole or lansoprazole.

A particularly preferred DPP-4 inhibitor to be emphasized within the meaning of this invention is 1-[(4-methyl-quinazolin-2-yl)methyl]-3-methyl-7-(2-butyn-1-yl)-8-(3-(*R*)-amino-piperidin-1-yl)-xanthine free base (also known as Bl 1356).

The present invention is not to be limited in scope by the specific embodiments described herein. Various modifications of the invention in addition to those described herein may become apparent to those skilled in the art from the present disclosure.

Further embodiments, features and advantages of the present invention may become apparent from the following examples. The following examples serve to further illustrate, by way of example, the principles of the invention without restricting it, and/or to aid in the understanding of the invention but are not construed as a limitation.

### Examples

### Experimental models for liver steatosis in rodents

### Animals and special diets

a) Female db/db mice [C57BLKS/Bom-db/db; Charles-River, Germany], aged 6-7 weeks at start are treated 8 weeks with the DPP-4 inhibitor. Animals are kept in groups of 5-6 mice in Makrolon® cages with free access to food and tap water. The room is maintained as follows: dark light rhythm [6 a.m. to 6 p.m. light], room temperature 22 ± 2 °C, relative humidity 50 ± 10%.
   The DPP-4 inhibitor is suspended in 0.5 % [w/v] natrosol. Single daily oral administrations of 3 mg/kg/d of the DPP-4 inhibitor in a volume of 5 ml/kg by gavage are performed. Control animals receive 0.5 % [w/v] natrosol, also in a volume of 5 ml/kg. At the end of the treatment period [8 weeks after treatment start] animals are dissected and liver biopsies taken.
b) Male ZDF rats (ZDF/Crl-lepr^{fa}), age 8-10 weeks at start are treated daily with the DPP-4 inhibitor for 35 days in a dose of 3 mg/kg /d. The application volume is 3 ml/kg natrosol. Animal (n=8-10) are houses in single cages. The room is maintained as follows: dark light rhythm [6 a.m. to 6 p.m. light], room temperature 22 ± 2 °C, relative humidity 50 ± 10 %.
c) Diet induced steatosis: Male C57BL/6 mice [C57BL/6NC] supplied by Charles River, Germany] are fed with a high fat diet [D12492, 60% kcal of fat, 5.24 kcal/g, Research diets, New Brunswick, USA] starting in the age of 9 weeks. Animals receive chow and water ad libitum. They are kept under controlled conditions and dark light rhythm [6 a.m. to 6 p.m. light]. Animals receive the high fat diet for 7 weeks before treatment start. Thereafter, animals are treated with the DPP-4 inhibitor (0.1 -10 mg/kg/d orally, suspended in 0.5 % natrosol or vehicle for 6 weeks once daily). Animals are sacrificed following an overnight fast and livers are removed for further analysis.
d) MCD induced steatosis: Male C57BL/6 mice [C57BL/6NC] supplied by Charles River, Germany] are allowed to normal diet or a MCD (methionine and cholin deficient diet) (Research Diets, New Brunswick, USA) and tap water ad libitum throughout the experimental period. At first, all mice are fed the normal diet during a 1-week quarantine and acclimation period. Then, at 9 weeks of age, mice displaying no abnormal findings at the end of the quarantine and acclimation period are randomly divided into groups (8-10 mice/group) and are treated for 5 weeks as follows: group 1 (normal), fed normal diet plus vehicle (0.5% [w/v] natrosol); group 2 (MCD control), fed MCD diet plus vehicle; group 3, fed MCD diet plus the DPP-4 inhibitor (3 mg/kg/day). At the end of the experimental period, blood samples are collected from the tail vein of the mice and the livers are collected. Histological analysis of inflammation and fibrosis are performed like under necropsy and histology.

### Detection of liver triglyceride content

Samples for hepatic triglycerides measurement are generated by homogenizing a liver lobe in lysis buffer (0,5% polyoxyethylen 10 tridecylether (Sigma, P 2393), 0.01 M NaPi, 1mM EDTA, pH 7.4). The liver tissue is previously heated in the same buffer 10 min to 95°C. Following the initial homogenization, samples are further processed using the MP Biotech (FastPrep-24) equipment and orange caps (2 x 45s). Samples are then centrifuged 2min at 3000rpm. The supernatant is collected for further analysis. Triglyceride content is measured using the Sigma serum triglyceride kit (Sigma, TR 0100).

### Necropsy and histology

Necropsy is performed in ad libitum fed animals. Animals are anesthetized with 3 % isoflurane / oxygen [Isoba®, batch J10417, Essex Pharma GmbH, 81737, Germany]. The liver is removed and fixed in 4 % paraformaldhyd solution for later microscopic. Liver lipidosis is evaluated blinded and semi-quantitatively by light microscopy. The slides are blindly examinated on coded slides and all parameters (e.g. steatosis, inflammation) are semi-quantitatively scored (severity index) by the following scheme:
Score 1 = no changes
Score 2 = minimal changes (< 5 % of liver tissues affected)
Score 3 = mild changes (5 -15 % of liver tissue affected)
Score 4 = moderate changes (15 - 25 % of liver tissue affected)
Score 5 = severe changes (> 25 % of liver tissue affected)

### Determination of liver steatosis by means of NMR spectroscopy

For the in vivo measurement of liver lipid content by NMR spectroscopy, C57BL/6 mice with diet-induced steatosis are anesthetised by continuous inhalation of 2 % isoflurane in a N₂O:O₂, (70:30, v:v) gas mixture. NMR measurements are performed on a Bruker BioSpec 47/40 scanner (Bruker BioSpin, Ettlingen, Germany) equipped with a BGA12 gradient coil system. A volume coil is used for excitation, a surface coil for signal reception. For anatomical orientation a pilot scan comprising horizontal and axial MR images is acquired using a gradient-echo pulse sequence with the following parameters: TR 135 ms, TE 3.5 ms, field-of view 45x45 mm², matrix 128², slice thickness 1.75 mm. According to the pilot scan, a voxel-of-interest (VOI, 3x3x3 mm³) is placed in the left ventral part of the liver. Liver lipids in the VOI are measured by NMR spectroscopy using a PRESS sequence (point-resolved spectroscopy) with the following parameters: TR 1050 ms, TE 20 ms, number of averages 32, digital resolution 1024 data points. Data are analyzed using the commercially available software package LCModel by S. W. Provencher.

In animal model c) described above (diet induced obesity mice) the following results are obtained for Bl 1356:

DIO mice 2 months on high fat diet (liver fat day 29 following treatment/control):

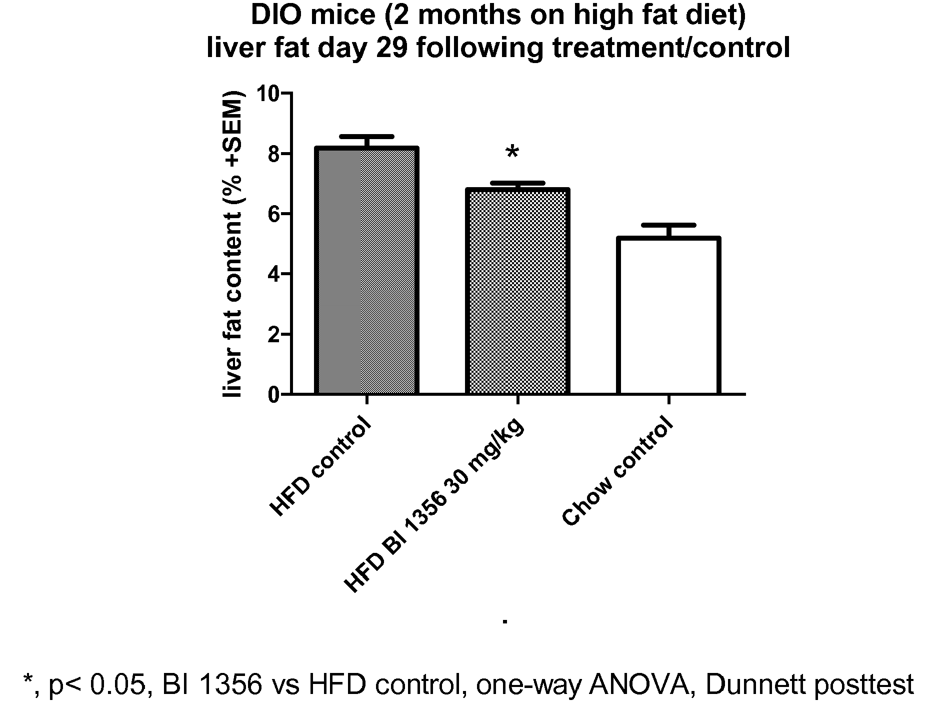

DIO mice 4 months on high fat diet (liver fat day 21 following treatment/control):

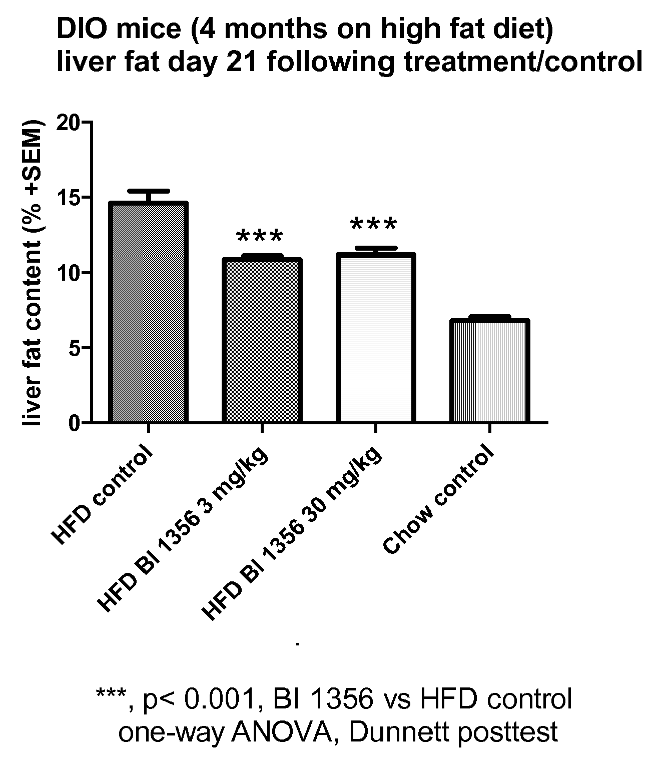

The effect on fatty liver diseases of the DPP-4 inhibitors of this invention can be tested in the models given herein and/or studied and detected by the methods described hereinabove and hereinbelow. The effect on various forms of fibrosis can be studied via the following detection methods:

### Methods for detection of fibrosis

### Quantitative measurements of specific mRNAs by RT-PCR

After disruption and homogenization in lysis buffer approximatively 50 mg snap frozen tissue, total RNA is isolated using a RNeasy Mini-kit (Qiagen, Germany). Transcripts for collagen type I, TGF-β1, procollagen α1(I), TIMP-1 (tissue inhibitor of matrix metalloproteinases-1), metalloproteases 1, 2, 3, 8, 9, 13 (MMP-1, MMP-2, MMP-3, MMP-8, MMP-9, MMP-13), PDGF-β receptor and α-smooth muscle cell actin are analysed via TQ-PCR and normalized to GAPDH mRNA.

### Sirius red staining of collagen

Sections 5 µm thick from formalin fixed, paraffin embedded tissue are prepared and are stained with Sirius red (saturated picric acid in distillated water containing 0.1% (w/v) Sirius red F3B (BDH Chemicals, UK) to allow visualisation of liver fibrosis. All samples from a series of experiments are stained simultaneously and evaluated in a blinded fashion.

### Necropsy and histology

Necropsy is performed in ad libitum fed animals. Animals are anesthetized with 3 % isoflurane / oxygen [Isoba®, batch J10417, Essex Pharma GmbH, 81737, Germany]. The organs (liver, kidney, lung, etc.) are removed and fixed in 4 % paraformaldhyd solution for later microscopic. Fibrosis is evaluated blinded and semi-quantitatively by light microscopy. The slides are blindly examinated on coded slides and all parameters are semi-quantitatively scored (severity index) by the following scheme¹:
Score 0 = no fibrosis
Score 1 = some portal tracts expanded
Score 2 = most portal tracts expanded
Score 3 = most portal tracts expended, +/- links
Score 4 = marked bridging (P-P and P-C links)
Score 5 = marked bridging, occasional nodules (incomplete cirrhosis)
Score 6 = cirrhosis, probably or definite
¹) Ishak etal. J Hepatol 22: 696, 1995

### Determination of fibrosis by means of Magnetic Resonance Elastography

Fibrosis is associated with increased stiffness and viscosity of the tissue. The viscoelastic properties of living tissue can be assessed with Magnetic Resonance Elastography (MRE) by measuring the propagation of low-frequency mechanical shear waves through the tissue. The propagation of the shear waves is visualized by non-invasive Magnetic Resonance Imaging (MRI), from which parameters for the mechanical properties of the tissue can be derived. Preclinical studies have shown that MRE can differentiate between the known stages of fibrosis and that the methods' results correspond with those of the gold-standard of biopsy².
²) Salameh et al. J. Magn. Reson. Imaging 26:956, 2007

### Effect on body weight

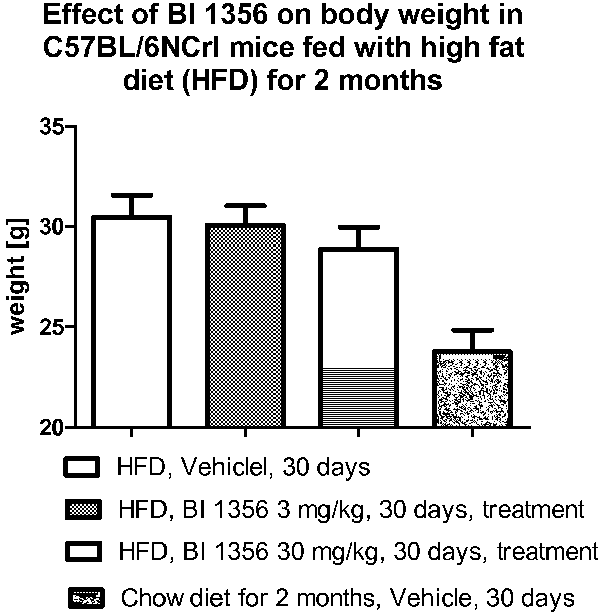

## Claims

1. A DPP-4 inhibitor of formula (I) 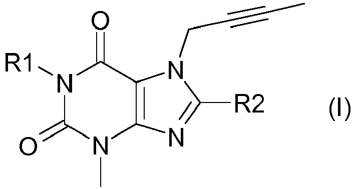 wherein **R1** denotes (4-methyl-quinazolin-2-yl)methyl, and **R2** denotes 3-(*R*)-amino-piperidin-1-yl, or its pharmaceutically acceptable salt;
for use in the treatment and/or prevention of non alcoholic fatty liver disease (NAFLD).

2. The DPP-4 inhibitor for use according to claim 1 wherein said DPP-4 inhibitor is 1-[(4-methyl-quinazolin-2-yl)methyl]-3-methyl-7-(2-butyn-1-yl)-8-(3-(*R*)-amino-piperidin-1-yl)-xanthine.

3. The DPP-4 inhibitor for use according to any of the claims 1 to 2 wherein said non alcoholic fatty liver disease (NAFLD) is selected from the group consisting of hepatic steatosis, non-alcoholic steatohepatitis (NASH) and liver fibrosis.

4. The DPP-4 inhibitor for use according to any of the claims 1 to 2, wherein the DPP-4 inhibitor is for use in the treatment and/or prevention of hepatic steatosis.

5. The DPP-4 inhibitor for use according to any of the claims 1 to 2, wherein the DPP-4 inhibitor is for use in the treatment and/or prevention of non-alcoholic steatohepatitis (NASH).

6. The DPP-4 inhibitor for use according to any of the claims 1 to 2, wherein the DPP-4 inhibitor is for use in the treatment and/or prevention of liver fibrosis.

7. A pharmaceutical composition for use in the treatment and/or prevention of non alcoholic fatty liver disease (NAFLD) including hepatic steatosis, non-alcoholic steatohepatitis (NASH) and/or liver fibrosis, said pharmaceutical composition comprising a DPP-4 inhibitor, which is 1-[(4-methyl-quinazolin-2-yl)methyl]-3-methyl-7-(2-butyn-1-yl)-8-(3-(*R*)-amino-piperidin-1-yl)-xanthine or a pharmaceutically acceptable salt thereof.

8. The pharmaceutical composition for use according to claim 7, said pharmaceutical composition further comprising metformin.

9. The pharmaceutical composition for use according to claim 7, said pharmaceutical composition further comprising pioglitazone.

10. Use of a DPP-4 inhibitor as defined in any of the claims 1 to 2 for the manufacture of a pharmaceutical composition for treatment and/or prevention of non alcoholic fatty liver disease (particularly diabetic NAFLD) including hepatic steatosis, non-alcoholic steatohepatitis (NASH) and/or liver fibrosis.

11. Use according to claim 10, wherein the pharmaceutical composition is for treating and/or preventing non alcoholic fatty liver disease (NAFLD)/non-alcoholic steatohepatitis (NASH), particularly NAFLD/NASH associated with diabetes.

12. The use according to claim 10 or 11, wherein the pharmaceutical composition further comprises one or more other active substances selected from the group consisting of antidiabetic substances, including active substances used to lower the blood sugar level, active substances used to lower the lipid level in the blood, active substances used to raise the HDL level in the blood, active substances used to lower blood pressure, active substances used for treating atherosclerosis, active substances used for treating obesity, antioxidants, and anti-inflammatory agents, such as e.g. for separate, sequential, simultaneous, concurrent or chronologically staggered use of the active ingredients.

13. A DPP-4 inhibitor according to any of the claims 1 to 2 for use in the treatment and/or prevention of non alcoholic fatty liver disease (NAFLD), including hepatic steatosis, non-alcoholic steatohepatitis (NASH) and/or liver fibrosis, in combination with one or more other active substances selected from the group consisting of antidiabetic substances, including active substances used to lower the blood sugar level, active substances used to lower the lipid level in the blood, active substances used to raise the HDL level in the blood, active substances used to lower blood pressure, active substances used for treating atherosclerosis, active substances used for treating obesity, antioxidants, and anti-inflammatory agents.

14. The use according to claim 12, wherein said other active substances are selected from the group consisting of biguanides including metformin, thiazolidinones including pioglitazone, statines including atorvastatin, and ARBs including telmisartan.

15. The DPP-4 inhibitor for use according to claim 13, wherein said other active substances are selected from the group consisting of biguanides including metformin, thiazolidinones including pioglitazone, statines including atorvastatin, and ARBs including telmisartan.

16. A pharmaceutical package containing a pharmaceutical composition comprising a DPP-4 inhibitor, which is 1-[(4-methyl-quinazolin-2-yl)methyl]-3-methyl-7-(2-butyn-1-yl)-8-(3-(R)-amino-piperidin-1-yl)-xanthine or a pharmaceutically acceptable salt thereof, for use in treating and/or preventing non alcoholic fatty liver disease (NAFLD) including hepatic steatosis, non-alcoholic steatohepatitis (NASH) and/or liver fibrosis, optionally in combination with one, two or more other active substances selected from the group consisting of antidiabetic substances, including active substances used to lower the blood sugar level, active substances used to lower the lipid level in the blood, active substances used to raise the HDL level in the blood, active substances used to lower blood pressure, active substances used for treating atherosclerosis, active substances used for treating obesity, antioxidants, anti-inflammatory agents, and vascular endothelial protecting agents.

17. The DPP-4 inhibitor for use according to any of the claims 1 to 2, wherein the DPP-4 inhibitor is for use in treating and/or preventing NAFLD/NASH and/or diseases or disorders related or associated therewith, in patients having one or more disorders selected from NAFLD, metabolic syndrome, insulin resistance, IGT, IFG, overweight, obesity (particularly visceral and/or abdominal obesity), dyslipidemia (e.g. hyperlipidemia, hypertriglyceridemia and/or hypoHDLemia), diabetes (particularly type 2 diabetes), hypertension, hyperglycemia, hyperinsulinemia, hyperuricemia, severe sleep apnoe, polycystic ovary syndrome, and chronic hepatitis C infection.

## Patentansprüche

1. DPP-4-Inhibitor der Formel (I) 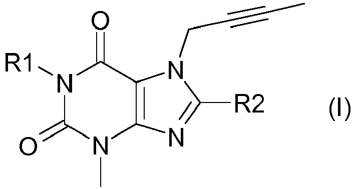 wobei **R1** (4-Methyl-chinazolin-2-yl)methyl darstellt, und **R2** 3-(*R*)-Amino-piperidin-1-yl darstellt, oder dessen pharmazeutisch akzeptables bzw. annehmbares Salz;
zur Verwendung in der Behandlung und/oder der Prävention von nichtalkoholischer Fettlebererkrankung (NAFLD).

2. DPP-4-Inhibitor zur Verwendung gemäß Anspruch 1, wobei besagter DPP-4-Inhibitor 1-[(4-Methyl-chinazolin-2-yl)methyl]-3-methyl-7-(2-butin-1-yl)-8-(3-(*R*)-amino-piperidin-1-yl)-xanthin ist.

3. DPP-4-Inhibitor zur Verwendung gemäß irgendeinem der Ansprüche 1 bis 2, wobei besagte nichtalkoholische Fettlebererkrankung (NAFLD) ausgewählt ist aus der Gruppe, bestehend aus Lebersteatose, nichtalkoholischer Steatohepatitis (NASH) und Leberfibrose.

4. DPP-4-Inhibitor zur Verwendung gemäß irgendeinem der Ansprüche 1 bis 2, wobei der DPP-4-Inhibitor in der Behandlung und/oder der Prävention von Lebersteatose verwendet wird.

5. DPP-4-Inhibitor zur Verwendung gemäß irgendeinem der Ansprüche 1 bis 2, wobei der DPP-4-Inhibitor in der Behandlung und/oder der Prävention von nichtalkoholischer Steatohepatitis (NASH) verwendet wird.

6. DPP-4-Inhibitor zur Verwendung gemäß irgendeinem der Ansprüche 1 bis 2, wobei der DPP-4-Inhibitor in der Behandlung und/oder der Prävention von Leberfibrose verwendet wird.

7. Pharmazeutische Zusammensetzung zur Verwendung in der Behandlung und/oder der Prävention von nichtalkoholischer Fettlebererkrankung (NAFLD) einschließlich Lebersteatose, nichtalkoholischer Steatohepatitis (NASH) und/oder Leberfibrose, besagte pharmazeutische Zusammensetzung umfassend einen DPP-4-Inhibitor, welcher 1-[(4-Methyl-chinazolin-2-yl)methyl]-3-methyl-7-(2-butin-1-yl)-8-(3-(R)-amino-piperidin-1-yl)-xanthin oder ein pharmazeutisch akzeptables bzw. annehmbares Salz davon ist.

8. Pharmazeutische Zusammensetzung zur Verwendung gemäß Anspruch 7, besagte pharmazeutische Zusammensetzung zusätzlich umfassend Metformin.

9. Pharmazeutische Zusammensetzung zur Verwendung gemäß Anspruch 7, besagte pharmazeutische Zusammensetzung zusätzlich umfassend Pioglitazon.

10. Verwendung eines wie in irgendeinem der Ansprüche 1 bis 2 definierten DPP-4-Inhibitors zur Herstellung einer pharmazeutischen Zusammensetzung zur Behandlung und/oder zur Prävention von nichtalkoholischer Fettlebererkrankung (insbesondere diabetische NAFLD) einschließlich Lebersteatose, nichtalkoholische Steatohepatitis (NASH) und/oder Leberfibrose.

11. Verwendung gemäß Anspruch 10, wobei die pharmazeutische Zusammensetzung zur Behandlung und/oder zur Prävention von nichtalkoholischer Fettlebererkrankung (NAFLD)/nichtalkoholischer Steatohepatitis (NASH), insbesondere NAFLD/NASH als Begleiterkrankung von Diabetes, bestimmt ist.

12. Verwendung gemäß Anspruch 10 oder 11, wobei die pharmazeutische Zusammensetzung zusätzlich ein oder mehrere andere aktive Substanzen umfasst, welche ausgewählt sind aus der Gruppe, bestehend aus antidiabetischen Substanzen, einschließlich aktiven Substanzen, welche zur Senkung des Blutzuckerspiegels verwendet werden, aktiven Substanzen, welche zur Senkung des Lipidspiegels im Blut verwendet werden, aktiven Substanzen, welche zur Erhöhung des HDL-Spiegel im Blut verwendet werden, aktiven Substanzen, welche zur Senkung des Blutdrucks verwendet werden, aktiven Substanzen, welche zur Behandlung von Atherosklerose verwendet werden, aktiven Substanzen, welche zur Behandlung von Adipositas verwendet werden, Antioxidantien und anti-inflammatorischen Mitteln, wie z.B. zur separaten, aufeinander folgenden, simultanen, gleichzeitigen oder zeitlich versetzten Verwendung der aktiven Bestandteile.

13. DPP-4-Inhibitor gemäß irgendeinem der Ansprüche 1 bis 2 zur Verwendung in der Behandlung und/oder der Prävention von nichtalkoholischer Fettlebererkrankung (NAFLD), einschließlich Lebersteatose, nichtalkoholischer Steatohepatitis (NASH) und/oder Leberfibrose, in Kombination mit einem oder mehreren anderen aktiven Substanzen, ausgewählt aus der Gruppe, bestehend aus antidiabetischen Substanzen, einschließlich aktiven Substanzen, welche zur Senkung des Blutzuckerspiegels verwendet werden, aktiven Substanzen, welche zur Senkung des Lipidspiegels im Blut verwendet werden, aktiven Substanzen, welche zur Erhöhung des HDL-Spiegel im Blut verwendet werden, aktiven Substanzen, welche zur Senkung des Blutdrucks verwendet werden, aktiven Substanzen, welche zur Behandlung von Atherosklerose verwendet werden, aktiven Substanzen, welche zur Behandlung von Adipositas verwendet werden, Antioxidantien und anti-inflammatorischen Mitteln.

14. Verwendung gemäß Anspruch 12, wobei besagten anderen aktiven Substanzen ausgewählt sind aus der Gruppe, bestehend aus Biguaniden einschließlich Metformin, Thiazolidinonen einschließlich Pioglitazon, Statinen einschließlich Atorvastatin und Angiotensinrezeptorblockern (ARB) einschließlich Telmisartan.

15. DPP-4-Inhibitor zur Verwendung gemäß Anspruch 13, wobei besagten anderen aktiven Substanzen ausgewählt sind aus der Gruppe, bestehend aus Biguaniden einschließlich Metformin, Thiazolidinonen einschließlich Pioglitazon, Statinen einschließlich Atorvastatin und Angiotensinrezeptorblockern (ARB) einschließlich Telmisartan.

16. Pharmazeutische Packung, enthaltend eine pharmazeutische Zusammensetzung, umfassend einen DPP-4-Inhibitor, welcher 1-[(4-Methyl-chinazolin-2-yl)methyl]-3-methyl-7-(2-butin-1-yl)-8-(3-(R)-amino-piperidin-1-yl)-xanthin oder ein pharmazeutisch akzeptables bzw. annehmbares Salz davon ist, zur Verwendung in der Behandlung und/oder der Prävention von nichtalkoholischer Fettlebererkrankung (NAFLD) einschließlich Lebersteatose, nichtalkoholischer Steatohepatitis (NASH) und/oder Leberfibrose, gegebenenfalls in Kombination mit einer, zwei oder mehreren anderen aktiven Substanzen, ausgewählt aus der Gruppe, bestehend aus antidiabetischen Substanzen, einschließlich aktiven Substanzen, welche zur Senkung des Blutzuckerspiegels verwendet werden, aktiven Substanzen, welche zur Senkung des Lipidspiegels im Blut verwendet werden, aktiven Substanzen, welche zur Erhöhung des HDL-Spiegel im Blut verwendet werden, aktiven Substanzen, welche zur Senkung des Blutdrucks verwendet werden, aktiven Substanzen, welche zur Behandlung von Atherosklerose verwendet werden, aktiven Substanzen, welche zur Behandlung von Adipositas verwendet werden, Antioxidantien, anti-inflammatorischen Mitteln und Mitteln, die das Gefäßendothel schützen.

17. DPP-4-Inhibitor zur Verwendung gemäß irgendeinem der Ansprüche 1 bis 2, wobei der DPP-4-Inhibitor zur Behandlung und/oder zur Prävention von NAFLD/NASH und/oder von damit verwandten oder verbundenen Erkrankungen oder Störungen verwendet wird, bei Patienten, die an einer oder mehreren Störungen ausgewählt aus NAFLD, metabolischem Syndrom, Insulinresistenz, gestörter Glukosetoleranz (IGT), gestörter Nüchternglukose (IFG), Übergewicht, Adipositas (insbesondere viszerale und/oder abdominale Adipositas), Dyslipidämie (e.g. Hyperlipidämie, Hypertriglyceridämie und/oder HypoHDLämie), Diabetes (insbesondere Typ 2 Diabetes), Bluthochdruck, Hyperglykämie, Hyperinsulinämie, Hyperurikämie, schwerer Schlaf-Apnoe, polyzystischem Ovarialsyndrom und chronischer Hepatitis C-Infektion leiden.

## Revendications

1. Inhibiteur de DPP-4 de formule (I) 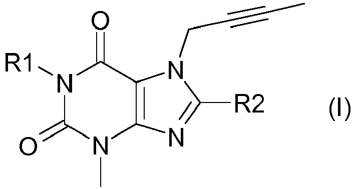 dans laquelle **R1** désigne (4-méthyl-quinazolin-2-yl)méthyle, and **R2** désigne 3-(*R*)-amino-pipéridin-1-yle, ou son sel pharmaceutiquement acceptable;
pour son utilisation dans le traitement et/ou la prévention de la stéato-hépatite non alcoolique (NAFLD).

2. Inhibiteur de DPP-4 pour son utilisation selon la revendication 1, ledit inhibiteur de DPP-4 inhibitor étant 1-[(4-méthyl-quinazolin-2-yl)méthyl]-3-éthyl-7-(2-butyn-1-yl)-8-(3-(*R*)-amino-pipéridin-1-yl)-xanthine.

3. Inhibiteur de DPP-4 pour son utilisation selon l'une quelconque des revendications 1 à 2, dans lequel ladite la stéato-hépatite non alcoolique (NAFLD) est choisie dans le groupe constitué par la stéatose hépatique, l'hépatite stéatosique non alcoolique (NASH) et la fibrose hépatique.

4. Inhibiteur de DPP-4 inhibitor pour son utilisation selon l'une quelconque des revendications 1 à 2, dans lequel l'inhibiteur de DPP-4 est à utiliser dans le traitement et/ou la prévention de la stéatose hépatique.

5. Inhibiteur de DPP-4 inhibitor pour son utilisation selon l'une quelconque des revendications 1 à 2, dans lequel l'inhibiteur de DPP-4 est à utiliser dans le traitement et/ou la prévention de l'hépatite stéatosique non alcoolique (NASH).

6. Inhibiteur de DPP-4 inhibitor pour son utilisation selon l'une quelconque des revendications 1 à 2, dans lequel l'inhibiteur de DPP-4 est à utiliser dans le traitement et/ou la prévention de la fibrose hépatique.

7. Composition pharmaceutique pour son utilisation dans le traitement et/ou la prévention de la stéato-hépatite non alcoolique (NAFLD) incluant la stéatose hépatique, l'hépatite stéatosique non alcoolique (NASH) et/ou la fibrose hépatique, ladite composition pharmaceutique comprenant un inhibiteur de DPP-4 qui est la 1-[(4-méthyl-quinazolin-2-yl)méthyl]-3-méthyl-7-(2-butyn-1-yl)-8-(3-(*R*)-amino-pipéridin-1-yl)-xanthine ou l'un de ses sels pharmaceutiquement acceptables.

8. Composition pharmaceutique pour son utilisation selon la revendication 7, ladite composition pharmaceutique comprenant en outre de la metformine.

9. Composition pharmaceutique pour son utilisation selon la revendication 7, ladite composition pharmaceutique comprenant en outre de la pioglitazone.

10. Utilisation d'un inhibiteur de DPP-4 tel que défini dans l'une quelconque des revendications 1 à 2, pour la fabrication d'une composition pharmaceutique destiné au traitement et/ou à la prévention de la stéato-hépatite non alcoolique (en particulier de la NAFLD diabétique) incluant la stéatose hépatique, l'hépatite stéatosique non alcoolique (NASH) et/ou la fibrose hépatique.

11. Utilisation selon la revendication 10, dans laquelle la composition pharmaceutique est destiné au traitement et/ou à la prévention de la stéato-hépatite non alcoolique (NAFLD)/ l'hépatite stéatosique non alcoolique (NASH), en particulier NAFLD/NASH associées au diabète.

12. Utilisation selon la revendication 10 ou 11, dans laquelle la composition pharmaceutique comprend en outre une ou plusieurs autres substances actives choisies dans le groupe constitué par des substances antidiabétiques, incluant des substances actives utilisées pour abaisser la glycémie, des substances actives utilisées pour abaisser la lipidémie dans le sang, des substances actives utilisées pour élever le taux de HDL dans le sang, des substances actives utilisées pour abaisser la tension artérielle, des substances actives utilisées pour traiter l'athérosclérose, des substances actives utilisées pour traiter l' obésité, des antioxydants, et des agents anti-inflammatoires, tels que par exemple pour l'utilisation séparée, séquentielle, simultanée, concomitante ou chronologiquement échelonnée des ingrédients actifs.

13. Inhibiteur de DPP-4 selon l'une quelconque des revendications 1 à 2 pour son utilisation dans le traitement et/ou la prevention de la stéato-hépatite non alcoolique (NAFLD), incluant la stéatose hépatique, l'hépatite stéatosique non alcoolique (NASH) et/ou la fibrose hépatique, en combinaison avec une ou plusieurs autres substances actives choisies dans le groupe constitué par des substances antidiabétiques, incluant des substances actives utilisées pour abaisser la glycémie, des substances actives utilisées pour abaisser la lipidémie dans le sang, des substances actives utilisées pour élever le taux de HDL dans le sang, des substances actives utilisées pour abaisser la tension artérielle, des substances actives utilisées pour traiter l'athérosclérose, des substances actives utilisées pour traiter l'obésité, des antioxydants et des agents anti-inflammatoires.

14. Utilisation selon la revendication 12, dans laquelle lesdites autres substances actives sont choisies dans le groupe constitué par les biguanides incluant la metformine, les thiazolidinones incluant la pioglitazone, les statines incluant l'atorvastatine et les ARB incluant le telmisartan.

15. Inhibiteur de DPP-4 pour son utilisation selon la revendication 13, dans laquelle lesdites autres substances actives sont choisies dans le groupe constitué par les biguanides incluant la metformine, les thiazolidinones incluant la pioglitazone, les statines incluant l'atorvastatine et les ARB incluant le telmisartan.

16. Emballage pharmaceutique contenant une composition pharmaceutique comprenant un inhibiteur de DPP-4, qui est la 1-[(4-méthyl-quinazolin-2-yl)méthyl]-3-méthyl-7-(2-butyn-1-yl)-8-(3-(R)-amino-pipéridin-1-yl)-xanthine ou l'un des ses sels pharmaceutiquement acceptables, pour son utilisation dans le traitement et/ou la prevention de la stéato-hépatite non alcoolique (NAFLD) incluant la stéatose hépatique, l'hepatite stéatosique non alcoolique (NASH) et/ou la fibrose hépatique, facultativement en combinaison avec une, deux ou plusieurs autres substances actives choisies dans le groupe constitué par des substances antidiabétiques, incluant des substances actives utilisées pour abaisser la glycémie, des substances actives utilisées pour abaisser la lipidémie dans le sang, des substances actives utilisées pour élever le taux de HDL dans le sang, des substances actives utilisées pour abaisser la tension artérielle, des substances actives utilisées pour traiter l'athérosclérose, des substances actives utilisées pour traiter l'obésité, des antioxydants, des agents anti-inflammatoires et des agents de protection endothéliale vasculaire.

17. Inhibiteur de DPP-4 pour son utilisation selon l'une quelconque des revendications 1 à 2, dans laquelle l'inhibiteur de DPP-4 est destiné pour l'utilisation dans le traitement et/ou la prévention de NAFLD/NASH et/ou des maladies ou troubles apparentés ou associés à celles-ci, dans les patients ayant une ou plusieurs des troubles choisis parmi NAFLD, syndrome métabolique, résistance d'insuline, intolérance au glucose (IGT), perturbation de la glycémie à jeun (IFG), surpoids, obésité (en particulier obésité viscérale et/ou abdominale), dyslipidémie (par exemple hyperlipidémie, hypertriglycéridémie et/ou hypoHDLémie), diabète (en particulier diabète de type 2), hypertension, hyperglycémie, hyperinsulinémie, hyperuricémie, apnée du sommeil sévère, syndrome ovarien polycystique et infection par le virus de l'hépatite C chronique.
